# EUROPEAN PATENT APPLICATION

(11) **EP 1 849 399 A1**
(43) Date of publication of application: **31.10.2007**
(21) Application number: 07251688.3
(22) Date of filing: 23.04.2007
(51) Int. Cl.: A61B 1/005, A61M 25/01

(54) **Medical cannula and medical cannula system**

(30) Priority: 24.04.2006 US 409720
(71) Applicant: ETHICON ENDO-SURGERY, INC., Cincinnati, Ohio 45242 (US)
(72) Inventor: Lu, Ifung, Cincinnati, Ohio 45227 (US); Nobis, Rudolph H., Mason, Ohio 45040 (US)
(74) Representative: Tunstall, Christopher Stephen

(57) **Abstract**

A medical cannula includes a first medical cannula tube, a second medical cannula tube, a resiliently bendable coil spring, and a flexible first elongate member. The first tube has a distal end portion insertable within a patient. The second tube is entirely insertable within the patient, is spaced apart from the first tube, and has a proximal end portion. The coil spring connects the first tube to the second tube. The first elongate member is located in the first tube, has a distal end portion extending from the first tube and attached to the second tube, and is movable to articulate the second tube with respect to the first tube. A medical cannula system additionally includes a medical instrument which is adapted to be passed through the first tube and into the second tube.

## Description

**Field of the Invention**

The present invention is related generally to medical equipment, and more particularly to a medical cannula and to a medical cannula system.

**Background of the Invention**

Endoscopes (including colonoscopes) are known which have an insertion tube which is insertable within a patient. The insertion tube has an articulatable distal end portion controlled by wires running from the distal end portion to control knobs on the handle of the endoscope. A wide angle video camera in the distal end of the insertion tube permits medical observation. Medical instruments, such as a snare, are part of an endoscopic system and are insertable into the working channel(s) of the insertion tube of the endoscope and are translatable to extend from the distal end portion for medical treatment.

Still, scientists and engineers continue to seek improved medical cannulas and medical cannula systems.

**Summary of the Invention**

A first expression of an embodiment of the invention is for a medical cannula including a first medical cannula tube, a second medical cannula tube, a resiliently bendable coil spring, and a flexible first elongate member. The first medical cannula tube has a distal end portion insertable within a patient. The second medical cannula tube is entirely insertable within the patient, is spaced apart from the first medical cannula tube, and has a proximal end portion. The coil spring connects the first medical cannula tube to the second medical cannula tube. The first elongate member is located in the first medical cannula tube, has a distal end portion extending from the first medical cannula tube and attached to the second medical cannula tube, and is movable to articulate the second medical cannula tube with respect to the first medical cannula tube.

A second expression of an embodiment of the invention is for a medical cannula system including a medical cannula as described in the previous paragraph and including a medical instrument. The medical instrument is adapted to be passed through the first medical cannula tube and into the second medical cannula tube when the second medical cannula tube and the distal end portion of the first medical cannula tube are located within the patient. The medical instrument is adapted to be carried with the second medical cannula tube when the first elongate member is moved to articulate the second medical cannula tube with respect to the first medical cannula tube.

Several benefits and advantages are obtained from one or more of the expressions of an embodiment of the invention. In one example, the first medical cannula tube is an endoscope end cap which is attachable to the distal end of a flexible insertion tube of an endoscope, wherein the second medical cannula tube can be articulated with respect to the first medical cannula tube (and hence with respect to the insertion tube of the endoscope) allowing independent alignment of the wide angle video camera of the endoscope and a medical instrument carried in the second medical cannula tube. In a second example, the first medical cannula tube has a cannula-tube-to-endoscope-rail coupling feature allowing the first medical cannula tube to be coupled to, and slid along, an exterior rail of a flexible insertion tube of an endoscope which also allows independent alignment of the wide angle video camera of the endoscope and a medical instrument carried in the second medical cannula tube.

The following is a non-exhaustive list of embodiments of the invention that may be claimed in this application or in subsequently filed divisional applications.

Embodiment 1. A medical cannula comprising: a) a first medical cannula tube having a distal end portion insertable within a patient; b) a second medical cannula tube entirely insertable within the patient, spaced apart from the first medical cannula tube, and having a proximal end portion; c) a resiliently bendable coil spring connecting the first medical cannula tube to the second medical cannula tube; and d) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube.

Embodiment 2. The medical cannula of embodiment 1, wherein the moved first elongate member is at least one of a pulled elongate member, a pushed elongate member, and a twisted elongate member to articulate the second medical cannula tube with respect to the first medical cannula tube.

Embodiment 3. The medical cannula of embodiment 1, wherein the first medical cannula tube has a proximal end portion, and wherein the first elongate member extends outside the proximal end portion of the first medical cannula tube.

Embodiment 4. The medical cannula of embodiment 1, also including a flexible second elongate member which is disposed in the first medical cannula tube and which has a distal end portion extending from the first medical cannula tube and attached to the second medical cannula tube, wherein the first elongate member and the second elongate member are circumferentially spaced apart at the second medical cannula tube.

Embodiment 5. The medical cannula of embodiment 1, wherein the first medical cannula tube is an endoscope end cap.

Embodiment 6. The medical cannula of embodiment 1, wherein the first medical cannula tube has a cannula-tube-to-endoscope-rail coupling feature.

Embodiment 7. The medical cannula of embodiment 1, wherein the coil spring is more bendable than the second medical cannula tube.

Embodiment 8. The medical cannula of embodiment 1, wherein the first and second medical cannula tubes and the coil spring are substantially coaxially aligned when the coil spring is in a relaxed state.

Embodiment 9. The medical cannula of embodiment 1, wherein the coil spring has contacting coil turns.

Embodiment 10. The medical cannula of embodiment 9, wherein the coil spring provides the only connection of the first medical cannula tube to the second medical cannula tube.

Embodiment 11. The medical cannula of embodiment 1, wherein the coil spring has non-contacting coil turns.

Embodiment 12. The medical cannula of embodiment 11, also including a spine rigidly connected to the distal end portion of the first medical cannula tube and pivotally connected to the proximal end portion of the second medical cannula tube.

Embodiment 13. The medical cannula of embodiment 12, wherein the spine is spaced apart from, and disposed outside of, the coil spring.

Embodiment 14. A medical cannula system comprising: a) a medical cannula including: (1) a first medical cannula tube having a distal end portion insertable within a patient; (2) a second medical cannula tube entirely insertable within the patient, spaced apart from the first medical cannula tube, and having a proximal end portion; (3) a resiliently bendable coil spring connecting the first medical cannula tube to the second medical cannula tube; and (4) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube; and b) a medical instrument adapted to be passed through the first medical cannula tube and into the second medical cannula tube when the second medical cannula tube and the distal end portion of the first medical cannula tube are disposed within the patient, wherein the medical instrument is adapted to be carried with the second medical cannula tube when the first elongate member is moved to articulate the second medical cannula tube with respect to the first medical cannula tube.

Embodiment 15. The medical cannula system of embodiment 14, wherein the medical instrument is a needle-knife.

Embodiment 16. The medical cannula of embodiment 14 wherein the first medical cannula tube is an endoscope end cap.

Embodiment 17. The medical cannula of embodiment 14, wherein the first medical cannula tube has a cannula-tube-to-endoscope-rail coupling feature.

Embodiment 18. A medical cannula comprising: a) a first medical cannula tube having a distal end portion insertable within a patient; b) a second medical cannula tube entirely insertable within the patient, spaced apart from the first medical cannula tube, and having a proximal end portion; c) a resiliently bendable coil spring connecting the first medical cannula tube to the second medical cannula tube; d) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube; and e) a flexible second elongate member which is disposed in the first medical cannula tube and which has a distal end portion extending from the first medical cannula tube and attached to the second medical cannula tube, wherein the first elongate member and the elongate member are circumferentially spaced apart at the second medical cannula tube, wherein the coil spring is more bendable than the second medical cannula tube, and wherein the first and second medical cannula tubes and the coil spring are substantially coaxially aligned when the coil spring is in a relaxed state.

Embodiment 19. The medical cannula of embodiment 18 wherein the first medical cannula tube is an endoscope end cap.

Embodiment 20. The medical cannula of embodiment 18, wherein the first medical cannula tube has a cannula-tube-to-endoscope-rail coupling feature.

The present invention has, without limitation, application in hand-activated instruments as well as in robotic-assisted instruments.

**Brief Description of the Figures**

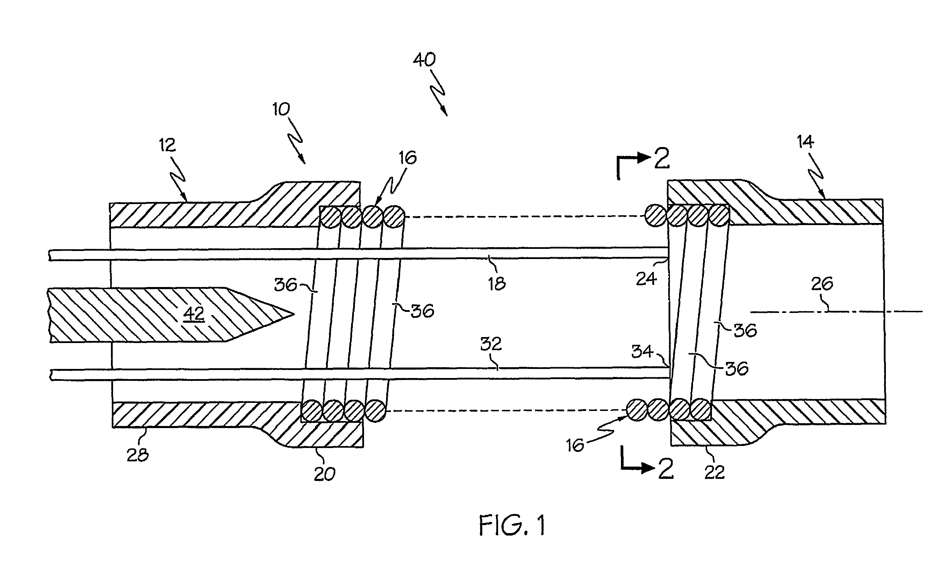

FIGURE 1 is a schematic, top elevational, cross sectional view of an embodiment of the invention including a medical cannula having a coil spring with contacting coil turns and including a medical instrument in the form of a needle-knife which is adapted to be insertable into the medical cannula, wherein the first medical cannula tube is an endoscope end cap;

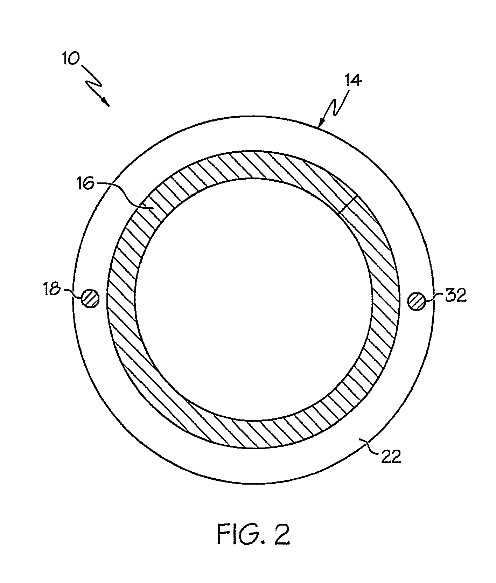

FIGURE 2 is view, taken along lines 2-2 in figure 1, showing that the elongate members of the medical cannula of figure 1 are circumferentially spaced apart; and

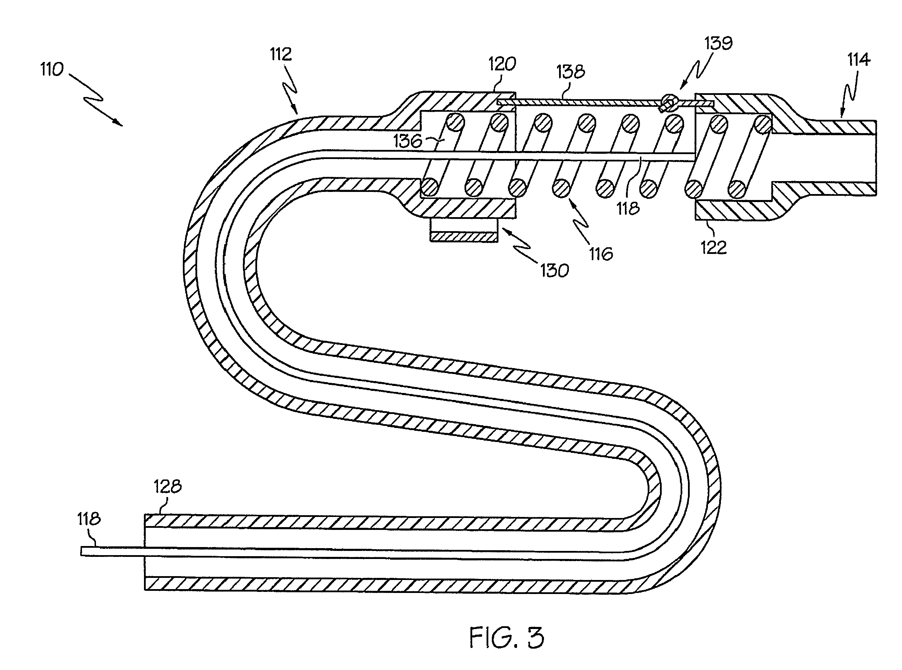

FIGURE 3 is a schematic, side elevational, cross sectional view of an alternate embodiment of the medical cannula of figure 1, wherein the medical cannula has a coil spring with non-contacting coil turns and has an optional spine, wherein the first medical cannula tube has a cannula-tube-to-endoscope-rail coupling feature.

**Detailed Description of the Invention**

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is understood that any one or more of the following-described embodiments, examples, etc. can be combined with any one or more of the other following-described embodiments, examples, etc.

Referring now to the Figures, in which like numerals indicate like elements, Figures 1-2 illustrate an embodiment of the invention. A first expression of the embodiment of Figures 1-2 is for a medical cannula 10 including a first medical cannula tube 12, a second medical cannula tube 14, a resiliently bendable coil spring 16, and a flexible first elongate member 18. The first medical cannula tube 12 has a distal end portion 20 insertable (such as endoscopically insertable) within a patient. The second medical cannula tube 14 is entirely insertable within the patient, is spaced apart from the first medical cannula tube 12, and has a proximal end portion 22. The coil spring 16 connects the first medical cannula tube 12 to the second medical cannula tube 14. The first elongate member 18 is located in the first medical cannula tube 12, has a distal end portion 24 extending from the first medical cannula tube 12 and attached to the second medical cannula tube 14, and is movable to articulate the second medical cannula tube 14 with respect to the first medical cannula tube 12.

It is noted that the terminology "elongate member" includes, without limitation, a wire such as a braided wire, a monolithic wire, or a wire having two or more wire segments with adjacent segments lengthwise joined together (i.e., one end of one segment is joined to one end of another segment). It is noted that a monolithic wire is a wire made of one continuous piece. In one choice of materials, the wire comprises, consists essentially of, or consists of nitinol. Other examples of elongate members are left to the artisan. In one configuration, the first elongate member 18 is disposed within the cannula lumen as shown in Figure 1. In another configuration, not shown, the first elongate member is disposed within the wall of the first medical cannula tube.

In one enablement of the first expression of the embodiment of Figures 1-2, the moved first elongate member 18 is at least one of a pulled elongate member, a pushed elongate member, and a twisted elongate member to articulate the second medical cannula tube 14 with respect to the first medical cannula tube 12. It is noted that the distal end portion 24 of the first elongate member 18 is attached to the second medical cannula tube 14 off axis from the longitudinal axis 26 of the second medical cannula tube 14.

In the same or a different enablement, the first medical cannula tube 12 has a proximal end portion 28, and the first elongate member 18 extends outside the proximal end portion 28 of the first medical cannula tube 12. In a first application, the first medical cannula tube 12 is an endoscope end cap. In the first application, the first medical cannula tube 12 is relatively short and, although not shown, is attachable to the distal end of a flexible insertion tube of an endoscope, wherein the first elongate member extends outside the proximal end portion of the first medical cannula tube and through a first working channel of the endoscope. In a second application, as shown in the alternate embodiment of Figure 3, the first medical cannula tube 112 has a cannula-tube-to-endoscope-rail coupling feature 130 (such as a "T" bar). In the second application, the first medical cannula tube 112 is relatively long and flexible and, although not shown, is attachable to an exterior rail (such as one having a "T"-shaped channel) of a flexible insertion tube of an endoscope and slidable along the rail toward the distal end of the insertion tube of the endoscope.

In the same or a different enablement, the medical cannula 10 also includes a flexible second elongate member 32 disposed in the first medical cannula tube 12 and having a distal end portion 34 extending from the first medical cannula tube 12 and attached to the second medical cannula tube 14. In one variation, the first elongate member 18 and the second elongate member 32 are circumferentially spaced apart at the second medical cannula tube 14 as seen in Figure 2. The number of elongate members and their circumferential angular separation are left to the artisan.

In one implementation of the first expression of the embodiment of Figures 1-2, the coil spring 16 is more bendable than the second medical cannula tube 14. In one variation, the second medical cannula tube 14 is a substantially rigid tube. In the same or a different implementation, the first and second medical cannula tubes 12 and 14 and the coil spring 16 are substantially coaxially aligned when the coil spring 16 is in a relaxed state.

In a first construction, the coil spring 16 has contacting coil turns 36. It is noted that the contacting coil turns 36 prevent axial compression of the coil spring 16 when, in one example, the first elongate member 18 is pulled to articulate the second medical cannula tube 14 with respect to the first medical cannula tube 12. In one variation, the coil spring 16 provides the only connection of the first medical cannula tube 12 to the second medical cannula tube 14. In this variation, the first and second elongate members 18 and 32 are not connected to the first medical cannula tube 12.

In a second construction, as shown in the alternate embodiment of Figure 3, the coil spring 116 has non-contacting coil turns 136. In one variation, the medical cannula 110 also includes a spine 138 rigidly connected to the distal end portion 120 of the first medical cannula tube 112 and pivotally connected (such as through a hook and eye arrangement 139 or other arrangement) to the proximal end portion 122 of the second medical cannula tube 114. In one modification, the spine 138 is spaced apart from, and disposed outside of, the coil spring 116.

A second expression of the embodiment of Figures 1-2 is for a medical cannula system 40 including a medical cannula 10 as described in the first expression of the embodiment of Figures 1-2 in paragraph [0018] and including a medical instrument 42. The medical instrument 42 is adapted to be passed through the first medical cannula tube 12 and into the second medical cannula tube 14 when the second medical cannula tube 14 and the distal end portion 20 of the first medical cannula tube 12 are disposed within the patient. The medical instrument 42 is adapted to be carried with the second medical cannula tube 14 when the first elongate member 18 is moved to articulate the second medical cannula tube 14 with respect to the first medical cannula tube 12.

In one illustration of the second expression of the embodiment of Figures 1-2, the medical instrument 42 is a needle knife as shown in Figure 1. Other illustrations of medical instruments are left to the artisan.

It is noted that the enablements, implementations, constructions, etc. of the first expression of the embodiment of Figures 1-2, and the alternate embodiment of Figure 3, are equally applicable to the second expression of the embodiment of Figures 1-2.

In a first method employing a first example of the medical cannula 10, wherein the first medical cannula tube 12 is an endoscope end cap, the first medical cannula tube 12 is attached to the distal end of a flexible insertion tube of an endoscope (not shown) with, in this example, first and second elongate members 18 and 32 extending through a working channel of the insertion tube to either control knob(s) on the endoscope handle or a position to be manually grasped outside the patient. Then, the insertion tube is endoscopically inserted within a patient. The video camera of the endoscope is used to help guide the insertion tube to patient tissue (such as a polyp) requiring medical treatment. Then, a medical instrument (such as a snare, not shown) is passed through the first medical cannula tube 12 and into the second medical cannula tube 14. Then, one or both of the first and second elongate members 18 and 32 are pulled, pushed, and/or twisted either manually or by using the control knob(s) to articulatably position the medical instrument 42 for medical treatment. In one deployment, and referring to Figure 2, pulling both the first and second elongate members 18 and 32 articulates the second medical cannula tube 14 downward, pulling just the first elongate member 18 articulates the second medical cannula tube 14 to the left, and pulling just the second elongate member 32 articulates the medical cannula tube 14 to the right. Then, the medical instrument 42 is at least partially translated outside the distal end portion of the second medical cannula tube 14 and used to medically treat the patient tissue (such as having the snare excise the polyp). Then, the process is reversed to remove the medical instrument 42 from the second and first medical cannula tubes 14 and 12 and to withdraw the insertion tube from the patient.

In a second method employing a second example of the medical cannula 110, wherein the first medical cannula tube 112 has a cannula-tube-to-endoscope-rail coupling feature 130, the first medical cannula tube 112 is coupled to an exterior rail of a flexible insertion tube of an endoscope (not shown) with, in this example, first elongate member 118 extending out the proximal end portion 128 of the first medical cannula tube 112 to either cannula control knob(s) attached to the endoscope handle or a position to be manually grasped outside the patient. Then, the insertion tube is endoscopically inserted within a patient. The video camera of the endoscope is used to help guide the insertion tube to patient tissue (such as a polyp) requiring medical treatment. Then, a medical instrument (such as a snare) is passed through the first medical cannula tube 112 and into the second medical cannula tube 114. Then the first elongate member 118 is pulled, pushed, and/or twisted either manually or by using the control knob(s) to articulatably position the medical instrument for medical treatment. Then, the medical instrument is at least partially translated outside the distal end portion of the second medical cannula tube 114 and used to medically treat the patient tissue (such as having the snare excise the polyp). Then, the process is reversed to remove the medical instrument from the second and first medical cannula tubes 114 and 112 and to withdraw the insertion tube from the patient.

In a third example (not shown) of the medical cannula, the medical cannula looks like the medical cannula 110 of Figure 3 without the cannula-tube-to-endoscope-rail coupling feature, and such medical cannula is inserted within a patient without being attached to an endoscope. Other examples of the medical cannula, and methods involving their use, are left to those skilled in the art.

Several benefits and advantages are obtained from one or more of the expressions of an embodiment of the invention. In one example, the first medical cannula tube is an endoscope end cap which is attachable to the distal end of a flexible insertion tube of an endoscope, wherein the second medical cannula tube can be articulated with respect to the first medical cannula tube (and hence with respect to the insertion tube of the endoscope) allowing independent alignment of the wide angle video camera of the endoscope and a medical instrument carried in the second medical cannula tube. In a second example, the first medical cannula tube has a cannula-tube-to-endoscope-rail coupling feature allowing the first medical cannula tube to be coupled to, and slid along, an exterior rail of a flexible insertion tube of an endoscope which also allows independent alignment of the wide angle video camera of the endoscope and a medical instrument carried in the second medical cannula tube.

While the present invention has been illustrated by a description of several embodiments, it is not the intention of the applicants to restrict or limit the spirit and scope of the appended claims to such detail. Numerous other variations, changes, and substitutions will occur to those skilled in the art without departing from the scope of the invention. For instance, the medical cannula and medical cannula system of the invention have application in robotic assisted surgery taking into account the obvious modifications of such systems, components and methods to be compatible with such a robotic system. It will be understood that the foregoing description is provided by way of example, and that other modifications may occur to those skilled in the art without departing from the scope and spirit of the appended Claims.

## Claims

1. A medical cannula comprising:
a) a first medical cannula tube having a distal end portion insertable within a patient;
b) a second medical cannula tube entirely insertable within the patient, spaced apart from the first medical cannula tube, and having a proximal end portion;
c) a resiliently bendable coil spring connecting the first medical cannula tube to the second medical cannula tube; and
d) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube.

2. The medical cannula of claim 1, wherein the moved first elongate member is at least one of a pulled elongate member, a pushed elongate member, and a twisted elongate member to articulate the second medical cannula tube with respect to the first medical cannula tube.

3. The medical cannula of claim 1, wherein the first medical cannula tube has a proximal end portion, and wherein the first elongate member extends outside the proximal end portion of the first medical cannula tube.

4. The medical cannula of claim 1, also including a flexible second elongate member which is disposed in the first medical cannula tube and which has a distal end portion extending from the first medical cannula tube and attached to the second medical cannula tube, wherein the first elongate member and the second elongate member are circumferentially spaced apart at the second medical cannula tube.

5. The medical cannula of claim 1, wherein the first medical cannula tube is an endoscope end cap.

6. The medical cannula of claim 1, wherein the first medical cannula tube has a cannula-tube-to-endoscope-rail coupling feature.

7. The medical cannula of claim 1, wherein the coil spring is more bendable than the second medical cannula tube.

8. The medical cannula of claim 1, wherein the first and second medical cannula tubes and the coil spring are substantially coaxially aligned when the coil spring is in a relaxed state.

9. The medical cannula of claim 1, wherein the coil spring has contacting coil turns.

10. The medical cannula of claim 9, wherein the coil spring provides the only connection of the first medical cannula tube to the second medical cannula tube.

11. A medical cannula system comprising:
a) a medical cannula including:
(1) a first medical cannula tube having a distal end portion insertable within a patient;
(2) a second medical cannula tube entirely insertable within the patient, spaced apart from the first medical cannula tube, and having a proximal end portion;
(3) a resiliently bendable coil spring connecting the first medical cannula tube to the second medical cannula tube; and
(4) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube; and
b) a medical instrument adapted to be passed through the first medical cannula tube and into the second medical cannula tube when the second medical cannula tube and the distal end portion of the first medical cannula tube are disposed within the patient, wherein the medical instrument is adapted to be carried with the second medical cannula tube when the first elongate member is moved to articulate the second medical cannula tube with respect to the first medical cannula tube.

12. A medical cannula comprising:
a) a first medical cannula tube having a distal end portion insertable within a patient;
b) a second medical cannula tube entirely insertable within the patient, spaced apart from the first medical cannula tube, and having a proximal end portion;
c) a resiliently bendable coil spring connecting the first medical cannula tube to the second medical cannula tube;
d) a flexible first elongate member which is disposed in the first medical cannula tube, which has a distal end portion extending from the first medical cannula tube and attached to the second medical cannula tube, and which is movable to articulate the second medical cannula tube with respect to the first medical cannula tube; and
e) a flexible second elongate member which is disposed in the first medical cannula tube and which has a distal end portion extending from the first medical cannula tube and attached to the second medical cannula tube, wherein the first elongate member and the elongate member are circumferentially spaced apart at the second medical cannula tube,
wherein the coil spring is more bendable than the second medical cannula tube, and wherein the first and second medical cannula tubes and the coil spring are substantially coaxially aligned when the coil spring is in a relaxed state.
